(19) **Europäisches Patentamt**
**European Patent Office**
**Office européen des brevets**

(11) Veröffentlichungsnummer: **0 610 729 A1**

(12) # EUROPÄISCHE PATENTANMELDUNG

(21) Anmeldenummer: **94101211.4**

(22) Anmeldetag: **27.01.94**

(51) Int. Cl.⁵: **C07K 1/14**, C07K 3/18, B01D 15/08

(30) Priorität: **27.01.93 DE 4302163**

(43) Veröffentlichungstag der Anmeldung: **17.08.94 Patentblatt 94/33**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach (DE)**

(72) Erfinder: **Walter, Joachim, Dr.**
**Friedenslinde 4**
**D-88441 Mittelbiberach (DE)**
Erfinder: **Berthold, Wolfgang, Dr.**
**Friedrich-Ebert-Strasse 32**
**D-88400 Biberach (DE)**
Erfinder: **Maass, Dieter**
**Ropachweg 42**
**D-88400 Biberach (DE)**
Erfinder: **Nothelfer, Franz**
**Treutweg 11**
**D-88400 Biberach (DE)**

(54) Verfahren zur Reinigung von Proteinen und Peptiden aus lipidhaltigen Lösungen.

(57) Die Erfindung betrifft ein Verfahren zur Reinigung von Proteinen und Peptiden, die aus einem Lipide enthaltenden wässrigen Medium gewonnen werden, insbesondere zur Reinigung von Proteinen und Peptiden, die in einem Prozeßschritt einer nach Teilchengröße trennenden Filtrationstechnik, beispielsweise einer Ultrafiltration, unterworfen werden, wobei auf das Vorhandensein von Lipiden zurückzuführende nachteilige Effekte bei der Anwendung der Filtrationstechnik vermieden werden.

EP 0 610 729 A1

Die Erfindung betrifft ein Verfahren zur Reinigung von Proteinen und Peptiden, die aus einem Lipide enthaltenden wässrigen Medium gewonnen werden, insbesondere zur Reinigung von Proteinen und Peptiden, die in einem Prozeßschritt einer nach Teilchengröße trennenden Filtrationstechnik unterworfen werden.

Im "downstream-processing" zur Herstellung pharmazeutischer Proteine und Peptide wird üblicherweise eine Abfolge von hintereinander durchzuführenden Reinigungsschritten angewandt, welche unter anderem nach Teilchengröße trennende Filtrationstechniken wie Mikro- oder Ultrafiltrationen unter Ausnutzung selektiver Porengrößen, z.B. zur Abtrennung viraler Verunreinigungen, sowie chromatographische Verfahren, basierend auf verschiedenen physikalisch-chemischen Eigenschaften von Protein-Molekülen, einschließen.

Durch die Anwesenheit von Lipiden im Kulturmedium bei der biotechnologischen Herstellung von Proteinen und Peptiden, bedingt entweder durch den Zusatz von Serum, von gereinigten lipidhaltigen Serumkomponenten mit definierter Zusammensetzung oder den Zusatz definierter Lipid-Gemische, beispielsweise eines wäßrigen Lipid-Zusatzes wie EX-CYTE[(R)] der Firma Bayer Diagnostics (vgl. auch L.J. Guilbert, N.N. Iscore, Nature 263, 594 (1976) und N.N. Iscore, F. Melchers, J. Exp. Med. 147, 923 (1978)), können bei den obengenannten Filtrationstechniken Probleme auftreten. So wird z. B. durch eine infolge hydrophober Wechselwirkungen gebildeten Deck-Schicht auf bei Ultrafiltrationsmembranen, z.B. auf Polyethersulfon-Membranen, die effektive Porengröße der Membranen stark verkleinert, wodurch die Produktausbeute negativ beeinflußt wird, oder die Membranporen werden infolge der Lipid-Wechselwirkung für das gewünschte Produkt völlig undurchlässig. Dabei spielt dieser "Lipideffekt" eine umso größere Rolle, je kleiner die Porengröße der eingesetzten Membranen ist. Begegnet man diesem nachteiligen Effekt durch die Verwendung von Membranen mit einer größeren Porengröße, so sind diese insbesondere in der Anfangsphase kurz nach Beginn der Membranfiltration auch für Moleküle mit unerwünscht hohem Molekulargewicht oder für entsprechend große Partikel, wie z.B. Viren, durchlässig, da die Lipid-bedingte Einstellung einer stationären Gleichgewichts-Porengröße eine gewisse Zeit benötigt.

Von E.G. Bligh und W.J. Dyer wurde eine Methode zur Lipidextraktion aus tierischem Gewebe, speziell aus Fischen, mit Hilfe von Chloroform-Methanol-Wassergemischen beschrieben (vgl. Can. J. Biochem. Physiol., Vol. 37, 911-917 (1959). Eine optimale Lipidextraktion wird durch Homogenisieren des Gewebes mit einer Mischung aus Chloroform und Methanol erreicht, wobei sich mit dem im Gewebe enthaltenen Wasser eine monophasische Mischung ergibt. Anschließend kann das Homogenat mit Wasser und/oder Chloroform unter Bildung eines biphasischen Systems verdünnt werden, wobei die Chloroformphase die Lipide enthält und die Methanol-Wasser-Phase die Nichtlipide. Diese Methode ist jedoch zur Entfernung von Lipiden aus pharmazeutischen Proteinen nicht geeignet, da labile Proteine in Lösung unter den angegebenen Bedingungen denaturiert werden können.

In der EP 0 197 554 ist ein Verfahren zur Virus-Desaktivierung und Depyrogenisierung in Produkten, die aus tierischen oder menschlichen Körperflüssigkeiten gewonnenen werden, wie Blutfraktionen oder Blut-Proteinen, unter Verwendung von Amphiphilen, Hypochlorid, β-Propiolacton und organischen Lösungsmitteln beschrieben.

Aufgabe der vorliegenden Erfindung ist die Entwicklung eines Verfahrens zur Reinigung von Proteinen und Peptiden, die aus einem Lipide enthaltenden wässrigen Medium gewonnen werden, in dem die störenden Lipide ohne die Gefahr der Denaturierung labiler Proteine abgetrennt werden können und in dem die obengenannten Probleme bei der Anwendung nach Teilchengröße trennender Filtrationstechniken nicht auftreten.

Die Aufgabe der Erfindung wurde mit Hilfe eines Reinigungsschrittes gelöst, in welchem das im Rohprodukt enthaltene Protein oder Peptid auf einer festen Phase adsorbiert wird, die Lipide durch Waschung mit einer Mischung aus einem mit Wasser mischbaren organischen Lösungsmittel und einer physiologisch verträglichen Pufferlösung von der festen Phase entfernt werden und das Protein oder Peptid anschließend in einem lipidfreien Zustand eluiert wird. Unter der Voraussetzung, daß keine Denaturierungsgefahr für das zu reinigende Protein oder Peptid besteht, beispielsweise bei der Reinigung stark lipophiler Proteine wie z.B. typischer Membranproteine, kann zur Entfernung der Lipide von der festen Phase das mit Wasser mischbare organische Lösungsmittel auch alleine ohne Zusatz einer physiologisch verträglichen wässrigen Pufferlösung verwendet werden.

In einem bevorzugten Verfahren der vorliegenden Anmeldung wird das Protein nach der Eluierung von der festen Phase einer nach Teilchengröße trennenden Filtrationstechnik, beispielsweise einer Ultrafiltration, unterworfen. Hierbei kommt, abhängig vom Molekulargewicht des Proteins oder Peptids, bei der Durchführung der Ultrafiltration die Verwendung von Ultrafiltrationsmodulen wie Ultrafiltrationsmembranen, beispielsweise Kunststoffpolymer- oder Cellulosemembranen, oder Keramikmodulen mit einer nominellen Ausschlußgrenze zwischen 1 und 500 KD in Betracht, wobei die Ausschlußgrenze des Ultrafiltrationsmoduls größer als das Molekulargewicht des zu reinigenden Proteins oder Peptids ist. Dabei muß allerdings berücksichtigt werden, daß der eigentlich relevante Trennparameter bei Ultrafiltrationen nicht das nominelle Molekulargewicht des zu reinigenden Proteins oder Peptids, sondern die effektive volumetrische Größe bzw. der Stoke'sche Radius des Moleküls im jeweiligen Medium darstellt, welche infolge unterschiedlicher Hydratisierung in verschiedenen Puffermedien in gewissen Ausmaß variieren können. Für die Reinigung von Peptiden wird bevorzugt eine Membran mit einer nominellen Ausschlußgrenze zwischen 1 und 20 KD verwendet. Für die Reinigung von Immunglobulinen der Klassen A, D, E und G kommt bevorzugt ein Ultrafiltrationsmodul mit einer nominellen Ausschlußgrenze zwischen 150 und 350 KD, für die Reinigung von Fab-Fragmenten und rekombinanten Proteinen ähnlichen Molekulargewichtes, wie beispielsweise r-tPA, ein Ultrafiltrationsmodul mit einer nominellen Ausschlußgrenze zwischen 50 und 150 KD und für die Reinigung von Interferonen ein Ultrafiltrationsmodul mit einer nominellen Ausschlußgrenze zwischen 20 und 50 KD in Betracht.

Zur Entfernung der Lipide von der festen Phase kommt beispielsweise ein Alkohol mit 2 bis 5 Kohlenstoffatomen, Ethylenglykol, Diethylenglykol, Tetrahydrofuran, Dioxan, Aceton, Dimethylformamid, Dimethylsulfoxid oder Acetonitril, insbesondere jedoch Ethanol, n-Propanol, i-Propanol, n-Butanol, i-Butanol oder tert. Butanol in Betracht. Besonders bevorzugt sind n-Propanol und i-Propanol.

Das zur Entfernung der Lipide von der festen Phase eingesetzte Lösungsmittel wird bevorzugt in Mischung mit einer physiologisch verträglichen wässrigen Pufferlösung verwendet, wobei das Lösungsmittel in einem Volumenanteil von 5 bis 50%, vorzugsweise von 10 bis 25%, in der Pufferlösung enthalten ist. Es werden bevorzugt physiologisch verträgliche Pufferlösungen mit einem pH-Wert von 5 bis 9 und einer Ionenstärke von 0,01 bis 2,00 M eingesetzt. Beispielsweise können Acetat-, Bicarbonat-, Borat-, Citrat-, Maleat-, Phosphat-, Phthalat-, Succinat-, Glycin-, Lysin-, Histidin- oder Tris-(hydroxymethyl)-aminomethan-Puffer, deren Gemische oder eine beliebige andere physiologische Pufferlösung, unter deren Einfluß das zu reinigende Produkt auf der festen Matrix gebunden bleibt und die keine unerwünschten irreversiblen Veränderungen oder eine Modifizierung des Produktes bewirkt, verwendet werden.

Als feste Phase zur Adsorption des Proteins oder Peptids kommt jede chromatographische Matrix in Betracht, die das zu reinigende Produkt bei der Entfernung der Lipide durch Waschung mit einem der vorstehend erwähnten Lösungsmittel oder Lösungsmittel/Puffer-Gemische gebunden hält. Geeignete feste Phasen sind insbesondere Kationen- oder Anionenaustauscher, eine hydrophobe Matrix oder eine für das zu reinigende Protein oder Peptid spezifische Affinitätschromatographie-Matrix, z. B. Protein A oder Protein G für Immunglobuline oder Immunaffinitätsgromatographie mittels geeigneter Antikörper, sowie Lysin- oder Arginin-Sepharose für r-tPA.

Nach der Entfernung der Lipide wird die feste Phase mit einer der vorstehend erwähnten, jedoch von organischen Lösungsmitteln freien physiologisch verträglichen Pufferlösung gewaschen, um das organische Lösungsmittel von der festen Phase zu entfernen, bevorzugt jedoch mit derjenigen Pufferlösung, die auch bei der Entfernung der Lipide im vorhergehenden Schritt verwendet wurde. Das auf der festen Phase adsorbierte Protein oder Peptid wird anschließend nach an sich bekannten Methoden von der festen Phase eluiert, beispielsweise ebenfalls mit einer wie vorstehend erwähnten physiologisch verträglichen Pufferlösung, mit einem geeigneten desorbierenden Mittel, wie z. B. einer Lösung von Natriumchlorid, Calciumchlorid, Magnesiumchlorid, $\epsilon$-Aminocapronsäure oder Natriumcitrat in einem der vorstehend erwähnten physiologisch verträglichen Pufferlösungen, durch Veränderung des pH-Wertes des verwendeten Elutionsmittels oder durch Zugabe eines organischen Lösungsmittels zum verwendeten Elutionsmittel, wobei die vorstehend erwähnten Elutionsmethoden auch in Kombination angewendet werden können. Die Elution des auf der festen Phase adsorbierten Proteins oder Peptids erfolgt jedoch bevorzugt unter Verwendung einer wie vorstehend erwähnten physiologisch verträglichen Pufferlösung.

Das erfindungsgemäße Verfahren läßt sich insbesondere für die Reinigung von in einer lipidhaltigen wässrigen Lösung enthaltenen Proteinen oder Peptiden, die mit Hilfe rekombinanter Methoden oder der Hybridoma-Technik unter Zusatz von Serum, von gereinigten lipidhaltigen Serumkomponenten mit definierter Zusammensetzung oder von definierten Lipid-Gemischen zum Kulturmedium hergestellt werden, und die für eine Anwendung beim Menschen oder bei Tieren, beispielsweise für pharmazeutische, therapeutische oder diagnostische Zwecke in Frage kommen, verwenden. Dabei kommt jedes beliebige im Zusammenhang mit rekombinanten Arbeitstechniken oder der Hybridoma-Technik verwendbare lipidhaltige Kulturmedium in Frage, wobei als Lipid-Komponenten Lipide bovinen und nichtbovinen Ursprungs, beispielsweise Cholesterol, essentielle Fettsäuren, Lipoproteine (HDL, LDL und VLDL), Prostanoide, Triglyceride oder Phospholipide wie Lecithin, Lysolecithin, Sphingomyelin, Phosphatidylethanolamin und andere in Betracht kommen. Die Lipide können in reiner Form, als wässrige Emulsionen oder als Lösung, gegebenenfalls zusammen mit einem geeigneten Lösungsvermittler oder einer geeigneten Trägersubstanz, wie z. B. Albumin, dem Kulturmedium zugesetzt werden. Vorzugsweise werden kommerziell erhältliche LipidZusammensetzungen wie EX-CYTE[R] (Bayer Diagnostics) dem Kulturmedium zugesetzt, beispielsweise in einer Menge von 1 bis 5 ml pro l Kulturmedium.

Das Verfahren eignet sich vorzugsweise zur Reinigung von rekombinanten und nativen Proteinen oder Peptiden mit einem Molekulargewicht bis ca. 500 KD aus einem wie oben definierten Kulturmedium, wobei die Ausschlußgrenze des bei der Ultrafiltration verwendeten Ultrafiltrationsmoduls so auf das Molekulargewicht des zu reinigenden Proteins oder Peptids abgestimmt wird, daß der Stoke'sche Radius der zu reinigenden rekombinanten bzw. nativen Protein- oder Peptidmoleküle unter den verwendeten Pufferbedingungen ausreichend klein ist, daß das entsprechende Protein oder Peptid die Membranporen passieren kann.

Besonders bevorzugt wird das Verfahren zur Reinigung von Immunglobulinen der Klassen A, D, E und G, insbesondere jedoch von monoklonalen Antikörpern des Typs IgG2, wie beispielsweise des monoklonalen Antikörpers anti-ICAM, zur Reinigung von Interferonen, insbesondere jedoch zur Reinigung von Interferon $\omega$, und zur Reinigung von r-tPA oder Fab-Fragmenten aus einem lipidhaltigen wäßrigen Medium angewendet.

Des weiteren stellt die Erfindung auch ein Verfahren zur Herstellung eines Proteins oder Peptids auf biotechnologischem Weg, z. B. mit Hilfe rekombinanter Techniken oder der Hybridoma-Technik, durch Kultivierung von Zellen in einem geeigneten Kulturmedium unter Bedingungen, die für die Produktion des gewünschten Proteins oder Peptids durch die Zellen geeignet sind, zur Verfügung, wobei dem Kulturmedium entweder Serum oder statt dessen gereinigte lipidhaltige Serumkomponenten mit definierter Zusammensetzung oder definierte Lipid-Gemische zugegeben werden, das Protein oder Peptid entweder in das Kulturmedium exprimiert oder nach der Kultivierung aus den Zellen freigesetzt wird und durch mehrere Reinigungsschritte aus dem resultierenden Lipide und das Protein oder Peptid enthaltenden Medium gewonnen und in lipidfreiem Zustand isoliert wird, wobei das Protein oder das Peptid in mindestens einem der Reinigungsschritte einer nach Teilchengröße trennenden Filtrationstechnik unterworfen wird, dadurch gekennzeichnet, daß vor der Durchführung der letztgenannten Filtrationstechnik die Lipide von dem Protein oder Peptid abgetrennt werden und das Protein oder Peptid durch ein wie vorstehend beschriebenes Reinigungsverfahren isoliert wird.

Ein wichtiger Gesichtspunkt bei der Herstellung von Produkten aus Säugetierzellen ist das potentielle Risiko des Vorhandenseins von Verunreinigungen biologischen Ursprungs, wie beispielsweise Viren. Es ist deshalb sicherzustellen, daß entsprechende Verunreinigungen in einem für die Anwendung in vivo bestimmten Proteinprodukt inaktiviert oder aus dem Produkt entfernt werden. Das erfindungsgemäße Verfahren ermöglicht die Anwendung der zur Abtrennung des gewünschten Produktes von infektiösen viralen Partikeln bekanntermaßen geeigneten Methode der Ultrafiltration auch bei der Reinigung von Proteinen aus lipidhaltigen Medien, insbesondere aus lipidhaltigen wässrigen Kulturmedien zur Herstellung von Proteinen oder Peptiden mit Hilfe rekombinanter Techniken oder der Hybridoma-Technik, da die eingangs erwähnten auf Lipide zurückzuführenden nachteiligen Effekte bei Ultrafiltrationsprozessen durch eine der Ultrafiltration vorausgehende erfindungsgemäße Lipidentfernung vermieden werden. Zudem wird dadurch die Verwendung von Ultrafiltrationsmodulen mit kleineren Porengrößen, beispielsweise mit einer nominellen Ausschlußgrenze von 200 oder 150 KD ermöglicht, d. h. es können Moleküle oder Partikel abgetrennt werden, die in der volumetrischen Größe näher an der Molekülgröße des gewünschten Produktes liegen. Dementsprechend wird die Effektivität der Entfernung von Viren wesentlich verbessert.

Legende zu den Abbildungen in Anhang:

Figur 1:  Chromatographisches Profil (UV 280 nm) des monoklonalen Antikörpers anti-ICAM bei der Lipidentfernung entsprechend Beispiel 1 an S-Sepharose FF durch Waschung mit 10 mM Natriumcitrat: Isopropanol = 80:20 (v:v),
Elution: 50 mM Natriumcitrat, 100 mM NaCl, pH = 7,8
lineare Flußrate: 200 cm h$^{-1}$.

Figur 2:  Chromatographisches Profil (UV 280 nm) des monoklonalen Antikörpers anti-ICAM bei der Lipidentfernung entsprechend Beispiel 2 an Protein G-Sepharose FF durch Waschung mit 20 mM Natriumcitrat: Isopropanol = 80:20 (v:v),
Elution: 0,1 M Glycin/$H_3PO_4$, pH = 3,5
lineare Flußrate: 120 cm h$^{-1}$

Figur 3:

a) UV-Profil (280 nm) des Filtrats einer 300 KD Ultrafiltration des lipidfreien monoklonalen Antikörpers anti-ICAM (vollständige Permeation des Produkts durch die Membran).
b) UV-Profil (280 nm) des Filtrats einer 300 KD Ultrafiltration des lipidhaltigen monoklonalen Antikörpers anti-ICAM entsprechend Beispiel 3. Kurz nach Beginn des Ultrafiltrationsprozesses nimmt die Extinktion der Permeatflüssigkeit infolge einer für das Produkt undurchlässig werdenden Membran stark ab.

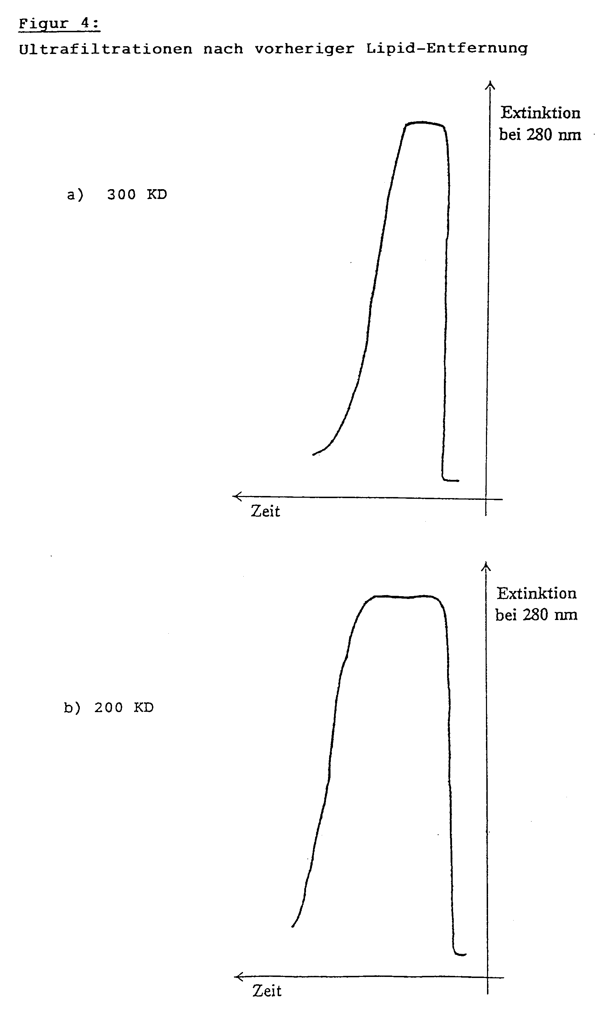

Figur 4: UV-Profile (280 nm) von Ultrafiltrationen des lipidfreien monoklonalen Antikörpers anti-ICAM entsprechend Beispiel 4.

Membran: Polyethersulfon, 350 cm$^2$

Konzentration: 12 mg ml$^{-1}$

 Puffer: Phosphatgepufferte Kochsalzlösung

Ausbeute: 95-100%

Nominelle Ausschlußgrenze:
  a) 300 KD
  b) 200 KD
  c) 150 KD

Die nachfolgenden Beispiele dienen der näheren Erläuterung der Erfindung.

Beispiel 1

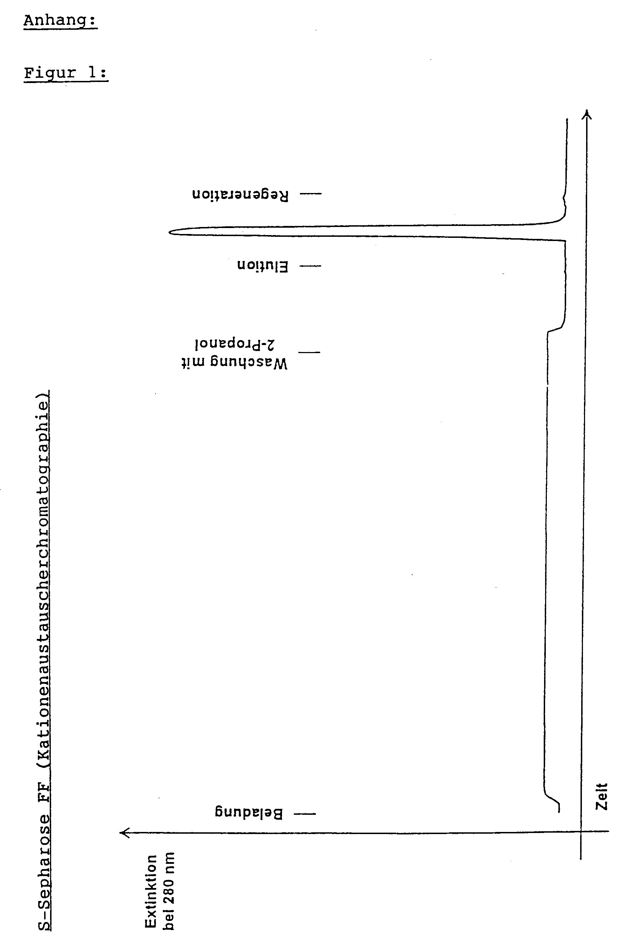

Das zum Zweck der Zellabtrennung mikrofiltrierte (0,1-0,2 $\mu$m) und auf einen für die Kationenaustauschchromatographie geeigneten pH-Wert und eine geeignete Leitfähigkeit adjustierte Rohprodukt des monoklonalen Antikörpers anti-ICAM, erhalten aus einem Kulturmedium, dem als Lipid-Zusatz 2,5 ml EX-CYTE$^{(R)}$ III (Bayer Diagnostics) pro l Medium zugegeben wurde, wird auf der zuvor mit einer wässrigen Pufferlösung von 10 mM Natriumcitrat (pH 6,25) äquilibrierten Kationenaustauschermatrix S-Sepharose FF (Pharmacia) adsorbiert (Säulendurchmesser 5 cm, Füllhöhe 10,3 cm). Nach der Beladung wird die Säule zur Entfernung der Lipide mit einer Mischung aus Äquilibrierungspuffer: Isopropanol = 80:20 (v:v) gewaschen. Danach wird erneut mit dem lösungsmittelfreiem Puffer äquilibriert und anschließend der Antikörper mit einer wässrigen Pufferlösung höherer Ionenstärke (50 mM Natriumcitrat, 100 mM NaCL, pH 7,8; lineare Flußrate 200 cm h$^{-1}$) eluiert. Die Matrix wird anschließend mit 1M NaOH regeneriert (vgl. Figur 1).

Beispiel 2

Das zum Zweck der Zellabtrennung mikrofiltrierte (0,1-0,2 $\mu$m) und auf einen für die Affinitätschromatographie geeigneten pH-Wert und eine geeignete Leitfähigkeit adjustierte Rohprodukt des monoklonalen Antikörpers anti-ICAM, erhalten aus einem Kulturmedium, dem als Lipid-Zusatz 2,5 ml EX-CYTE$^{(R)}$ III (Bayer Diagnostics) pro l Medium zugegeben wurde, wird auf der zuvor mit einer wässrigen Pufferlösung von 20 mM Natriumcitrat (pH 6,25) äquilibrierten Affinitätschromatographiematrix Protein G-Sepharose FF (Pharmacia) adsorbiert (Säulendurchmesser 18 cm, Füllhöhe 17 cm). Nach der Beladung wird die Säule zur Entfernung der Lipide mit einer Mischung aus Äquilibrierungspuffer: Isopropanol = 80:20 (v:v) gewaschen. Danach wird erneut mit dem lösungsmittelfreien Puffer äquilibriert und anschließend der Antikörper mit einer wässrigen Pufferlösung niedrigen pH-Wertes (0,1 M Glycin/H$_3$PO$_4$, pH 3,5; lineare Flußrate 120 cm h$^{-1}$) eluiert.

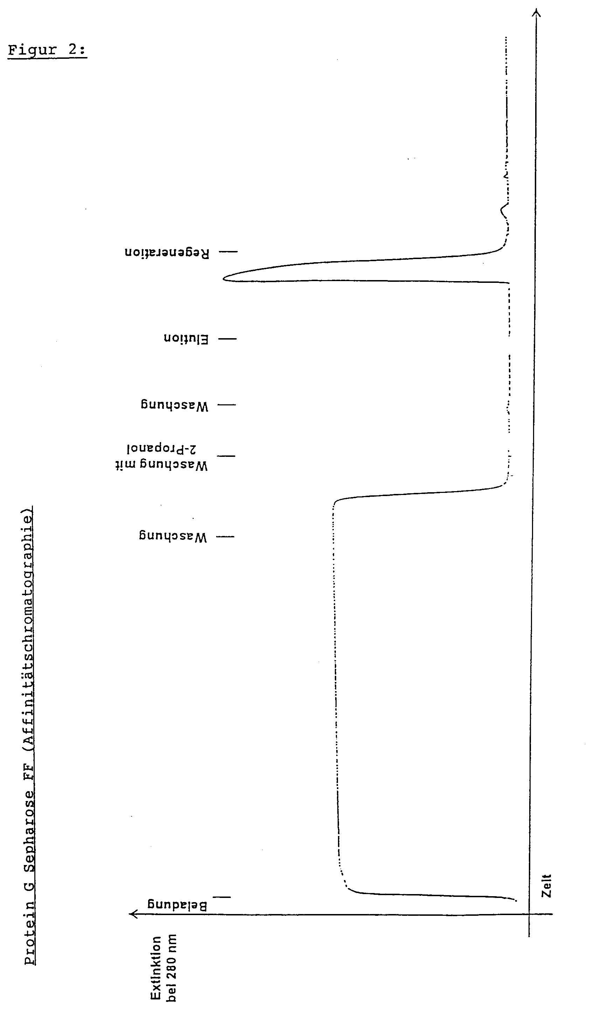

Die Matrix wird anschließend nacheinander mit wäßriger 1 M Essigsäure und 6 M Harnstofflösung regeneriert (vgl. Figur 2).

Die in den folgenden Beispielen 3 bis 5 beschriebenen Ultrafiltrationen des monoklonalen Antikörpers anti-ICAM wurden mittels Tangentialflußfiltration durchgeführt. Es wurden Membranen mit verschiedenen nominellen Ausschlußgrenzen eingesetzt und die Produkt-Ausbeute geprüft. Dabei wurden folgende Geräte verwendet:

Membranhalter "Minisette", Fabr. Filtron (USA)

Schlauchpumpe Watson Marlow 501 U

Membrankasetten:

Minisette 300 KD omega, 350 cm$^2$, Fabr. Filtron (USA)

Minisette 200 KD omega, 350 cm$^2$, Fabr. Filtron (USA)

Minisette 150 KD omega, 350 cm$^2$, Fabr. Filtron (USA)

In jedem Versuchsansatz wurde ein Probevolumen von 500 ml, in welcher das Protein mit einer Konzentration von 12 mg/ml enthalten war, durch die entsprechenden Membrankasetten ultrafiltriert. Als Puffer wurde Phosphat-gepufferte physiologische Kochsalzlösung verwendet. Der Eingangsdruck lag bei 0,5 bar, der Ausgangsdruck bei 0 bis 0,2 bar. Hieraus resultierte ein Transmembrandruck von 0,25 bis 0,35 bar:

|        | Eingangsdruck [bar] | Ausgangsdruck [bar] | Transmembrandruck [bar] |
|--------|---------------------|---------------------|-------------------------|
| 300 KD | 0,5                 | 0,0                 | 0,25                    |
| 200 KD | 0,5                 | 0,15                | 0,33                    |
| 150 KD | 0,5                 | 0,20                | 0,35                    |

Die Überströmrate lag bei 1,4 ml cm$^{-2}$ min$^{-1}$. Der Produkthaltige Filtratstrom wurde mittels UV-Meßzelle dedektiert und das entsprechende Filtrationsprofil mittels Schreiber dokumentiert.

Beispiel 3

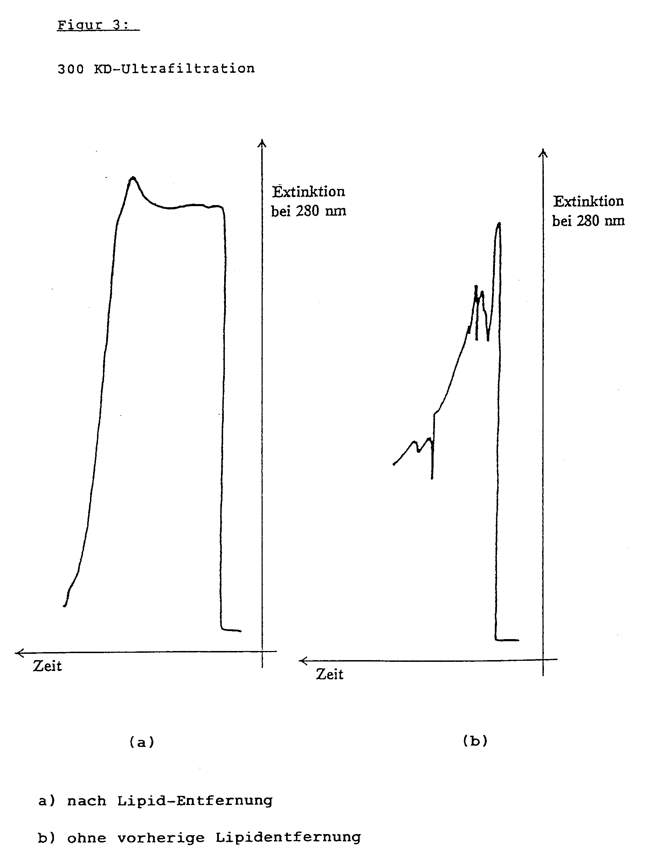

Ein analog Beispiel 1 vorbehandeltes Rohprodukt des monoklonalen Antikörpers anti-ICAM, bei dem jedoch kein Waschungsschritt mit Äquilibrierungspuffer:Isopropanol = 80:20 (v:v) durchgeführt wurde und welches aus diesem Grund noch Lipide enthält, wird unter Verwendung einer Membran mit einer nominellen Ausschlußgrenze von 300 KD ultrafiltriert. Bereits unmittelbar nach Beginn des Ultrafiltrationsprozesses nimmt die Extinktion stark ab, da das Produkt die Membranporen nicht mehr passiert (vgl. Figur 3b).

Beispiel 4

Das aus Beispiel 1 erhaltene lipidfreie Rohprodukt des monoklonalen Antikörpers anti-ICAM wird jeweils unter Verwendung einer Membran mit einer nominellen Ausschlußgrenze von 300 KD, 200 KD und 150 KD ultrafiltriert. Dabei nimmt der Permeatfluß mit der kleiner werdenden Ausschlußgrenze ab, d. h. die Prozeßdauer für die Gesamtfiltration nimmt entsprechend zu. In allen drei Fällen wird das Produkt jedoch nahezu quantitativ ultrafiltriert (vgl. Figur 4). Die Wiederfindungsrate im Filtrat beträgt für die 300 KD Membran 98 % und für die 200 KD und 150 KD Membran jeweils 100 %.

Beispiel 5

Mit Hilfe von Spiking-Experimenten (vgl.: Virus Removal and Inactivation, J.K. Walter, W. Werz, W. Berthold, Biotech Forum Europe Vol. 9, No. 9, 560-564, 1992; Downstream Processing von aus Zellkulturen gewonnenen Produkten, J.K. Walter, Bio Engineering 5, 14-19, 1990; Virus Removal/Inactivation in Downstream Processing, J.K. Walter, W. Werz, P. Mc Goff, R.G. Werner, W. Berthold, 11. ESACT meeting, Brighton, GB, September 2-6, 1991, veröffentlicht in: Animal Cell Technology: Developments, Process & Products, Editors R.E. Spier, J.B. Griffiths, C. MacDonald, Butterworth-Heinemann Ltd., Oxford, 1992, S. 624-634) wurde die Effektivität der Virus-Entfernung durch Ultrafiltration des nach Beispiel 1 lipidfrei erhaltenen Rohproduktes des monoklonalen Antikörpers anti-ICAM ermittelt. Als Testviren wurden das nichtumhüllte Virus Reo 3 und das umhüllte Parainfluenza 3 (PI-3)-Virus verwendet. Es wurden jeweils definierte Mengen einer entsprechenden Viruslösung mit bekannter Konzentration an infantiösen Viruspartikeln zu Proben des lipidfreien Rohproduktes des monoklonalen Antikörpers anti-ICAM gegeben und anschließend unter Verwendung von Membranen mit einer nominellen Anschlußgrenze von 300 KD und 200 KD ultrafiltriert. Für die 300 KD und 200 KD Ultrafiltration wurde die virale Abreicherung an jeweils 3 Proben bestimmt, wobei der gemessene Virus-Titer der Beladung jeweils mit dem gemessenen Titer des Filtrats verglichen wurde. In der folgenden Tabelle 1 ist der Abreicherungsfaktor in logarithmischer Form angegeben. Gegenüber der 300 KD Ultrafiltration läßt sich durch Verwendung einer Membran mit einer nominellen Ausschlußgrenze von 200 KD eine beachtliche Verbesserung der viralen Abreicherung erzielen.

Tabelle 1: Virusabreicherung durch 200 KD und 300 KD-Ultra-filtration

| Versuch | | Reo 3 | | | |
|---------|-------|-------|---------|---------|---------|
| nomi-nelle Ausschluß-grenze | Probe | Titer | Volumen | Gesamt-virus | Abreiche-rungs-faktor |
| | | $[\log \text{ml}^{-1}]$ | [ml] | [log] | [log] |
| 300 KD | 1.Beladung | 5.68 | 100.00 | 7.68 | |
| | 1.Filtrat | 2.68 | 164.56 | 4.89 | 2.79 |
| | 1.Retentat | 4.43 | 50.00 | 6.13 | |
| | 2.Beladung | 5.30 | 100.00 | 7.30 | |
| | 2.Filtrat | 2.30 | 163.35 | 4.51 | 2.79 |
| | 2.Retentat | 5.18 | 50.00 | 6.88 | |
| | 3.Beladung | 6.05 | 100.00 | 8.05 | |
| | 3.Filtrat | 3.30 | 160.73 | 5.51 | 2.54 |
| | 3.Retentat | 4.93 | 50.00 | 6.63 | |
| 200 KD | 1.Beladung | 5.93 | 100.00 | 7.93 | |
| | 1.Filtrat | n.d. | 163.32 | n.d. | 5.93 |
| | 1.Retentat | 5.05 | 50.00 | 6.75 | |
| | 2.Beladung | 6.18 | 100.00 | 8.18 | |
| | 2.Filtrat | n.d. | 157.55 | n.d. | 6.18 |
| | 2.Retentat | 5.05 | 50.00 | 6.75 | |
| | 3.Beladung | 5.68 | 100.00 | 7.68 | |
| | 3.Filtrat | <1.80 | 164.64 | <4.02 | >3.66 |
| | 3.Retentat | 5.30 | 50.00 | 6.99 | |

n.d. = nicht detektierbar

Fortsetzung Tabelle 1: Virusabreicherung durch 200 KD und 300 KD-Ultrafiltration

| Versuch | | PI-3 | | | |
|---|---|---|---|---|---|
| nomi-<br>nelle<br>Ausschluß-<br>grenze | Probe | Titer | Volumen | Gesamt-<br>virus | Abreiche-<br>rungs-<br>faktor |
| | | [log ml$^{-1}$] | [ml] | [log] | [log] |
| 300 KD | 1.Beladung | 6.30 | 100.00 | 8.30 | |
| | 1.Filtrat | 2.55 | 164.45 | 4.77 | 3.53 |
| | 1.Retentat | 6.30 | 50.00 | 7.99 | |
| | 2.Beladung | 6.43 | 100.00 | 8.43 | |
| | 2.Filtrat | <1.80 | 176.14 | <4.05 | >4.38 |
| | 2.Retentat | 6.05 | 50.00 | 7.75 | |
| | 3.Beladung | 6.68 | 100.00 | 8.68 | |
| | 3.Filtrat | 2.05 | 164.56 | 4.27 | 4.41 |
| | 3.Retentat | 6.05 | 50.00 | ·7.75 | |
| 200 KD | 1.Beladung | 6.68 | 100.00 | 8.68 | |
| | 1.Filtrat | <1.80 | 157.28 | <3.99 | >4.69 |
| | 1.Retentat | 6.18 | 50.00 | 7.88 | |
| | 2.Beladung | 6.80 | 100.00 | 8.80 | |
| | 2.Filtrat | n.d. | 148.74 | n.d. | 6.80 |
| | 2.Retentat | 6.93 | 50.00 | 8.63 | |
| | 3.Beladung | 6.93 | 100.00 | 8.93 | |
| | 3.Filtrat | n.d. | 158.86 | n.d. | 6.93 |
| | 3.Retentat | 6.68 | 50.00 | 8.38 | |

n.d. = nicht detektierbar

Protein: monoklonaler Antikörper anti-ICAM, 5 mg/ml

| | Reo 3 | PI-3 |
|---|---|---|
| Titer der Arbeitszellbank: | $10^{7.8}$ ml$^{-1}$ | $10^{7.18}$ ml$^{-1}$ |
| Verdünnungsfaktor der zugegebenen Viruslösung: | 1:10 | 1:10 |
| Infektiöse Viruspartikel: | $10^{6.8}$ ml$^{-1}$ | $10^{6.18}$ ml$^{-1}$ |

**Patentansprüche**

1. Verfahren zur Reinigung eines in einem lipidhaltigen wäßrigen Medium enthaltenen Proteins oder Peptids, dadurch gekennzeichnet, daß das im Rohprodukt enthaltene Protein oder Peptid auf einer festen Phase adsorbiert wird, die Lipide durch Waschung mit einem mit Wasser mischbaren organischen Lösungsmittel oder einer Mischung einer wäßrigen physiologisch verträglichen Pufferlösung mit einem mit Wasser mischbaren organischen Lösungsmittel von der festen Phase entfernt werden und anschließend das Protein oder Peptid in einem lipidfreien Zustand eluiert wird.

2. Verfahren gemäß Anspruch 1, dadurch gekennzeichnet, daß das Protein oder Peptid nach der Eluierung von der festen Phase einer nach Teilchengröße trennenden Filtrationstechnik unterworfen wird.

3. Verfahren gemäß Anspruch 2, dadurch gekennzeichnet, daß es sich bei der Filtrationstechnik um eine Ultrafiltration handelt und ein Ultrafiltrationsmodul mit einer nominellen Ausschlußgrenze zwischen 1 und 500 KD verwendet wird, wobei die nominelle Ausschlußgrenze des Ultrafiltrationsmoduls größer als das Molekulargewicht des zu reinigenden Proteins oder Peptids ist.

4. Verfahren gemäß Anspruch 3, dadurch gekennzeichnet, daß das Ultrafiltrationsmodul eine Ultrafiltrationsmembran ist.

5. Verfahren gemäß Anspruch 4, dadurch gekennzeichnet, daß das wäßrige Medium ein Peptid enthält und die Ultrafiltrationsmembran eine nominelle Ausschlußgrenze zwischen 1 und 20 KD besitzt oder das wäßrige Medium ein Immunglobulin der Klasse A, D, E oder G enthält und die Ultrafiltrationsmembran eine nominelle Ausschlußgrenze zwischen 150 und 350 KD besitzt oder das wäßrige Medium ein Fab-Fragment enthält und die Ultrafiltrationsmembran eine nominelle Ausschlußgrenze zwischen 50 und 150 KD besitzt oder das wäßrige Medium r-tPA enthält und die Ultrafiltrationsmembran eine nominelle Ausschlußgrenze zwischen 50 und 150 KD besitzt oder das wäßrige Medium ein Interferon enthält und die Ultrafiltrationsmembran eine nominelle Ausschlußgrenze zwischen 20 und 50 KD besitzt.

6. Verfahren gemäß einem der Ansprüche 1 bis 5, dadurch gekennzeichnet, daß das organische Lösungsmittel in einem Volumenanteil von 5 bis 50% in der physiologischen verträglichen Pufferlösung enthalten ist.

7. Verfahren gemäß einem der Ansprüche 1 bis 6, dadurch gekennzeichnet, daß das organische Lösungsmittel ein Alkohol mit 2 bis 5 Kohlenstoffatomen, Ethylenglykol, Diethylenglykol, Tetrahydrofuran, Dioxan, Aceton, Dimethylformamid, Dimethylsulfoxid oder Acetonitril ist.

8. Verfahren gemäß einem der Ansprüche 1 bis 7, dadurch gekennzeichnet, daß die physiologisch verträgliche Pufferlösung einen pH-Wert von 5 bis 9 und eine Ionenstärke von 0,01 bis 2,00 M besitzt und daß es sich um einen Acetat-, Bicarbonat-, Borat-, Citrat-, Maleat-, Phosphat-, Phthalat-, Succinat-, Glycin-, Lysin-, Histidin- oder Tris(hydroxymethyl)-aminomethan-Puffer oder deren Gemische handelt.

9. Verfahren gemäß einem der Ansprüche 1 bis 8, dadurch gekennzeichnet, daß als feste Phase zur Adsorption des Proteins oder Peptids eine chromatographische Matrix verwendet wird.

10. Verfahren gemäß einem der Ansprüche 1 bis 9, dadurch gekennzeichnet, daß das Protein oder Peptid auf biotechnologischem Weg hergestellt wird, wobei dem Kulturmedium Serum oder stattdessen gereinigte lipidhaltige Serumkomponenten oder definierte Lipid-Gemische zugegeben werden.

11. Verfahren gemäß einem der Ansprüche 1 bis 10, dadurch gekennzeichnet, daß das auf der festen Phase adsorbierte Protein oder Peptid mit einer physiologisch verträglichen Pufferlösung, einer Lösung von Natriumchlorid, Calciumchlorid, Magnesiumchlorid, $\epsilon$-Aminocapronsäure oder Natriumcitrat in einer physiologisch verträglichen Pufferlösung, durch Veränderung des pH-Wertes des Elutionsmittels, durch Zugabe eines organischen Lösungsmittels zum Elutionsmittel oder durch Erhöhung der Ionenstärke des Elutionsmittel von der festen Phase eluiert wird, wobei die Elutionsmethoden auch in Kombination angewendet werden können.

12. Verfahren zur Entfernung viraler Verunreinigungen aus einem in einem lipidhaltigen wäßrigen Medium enthaltenen Protein oder Peptid, dadurch gekennzeichnet, daß das im Rohprodukt enthaltene Protein oder Peptid auf einer festen Phase adsorbiert wird, die Lipide durch Waschung mit einem mit Wasser mischbaren organischen Lösungsmittel oder einer Mischung einer wäßrigen physiologisch verträglichen Pufferlösung mit einem mit Wasser mischbaren organischen Lösungsmittel von der festen Phase entfernt werden, das Protein oder Peptid in einem lipidfreien Zustand in einer wäßrigen physiologisch verträglichen Pufferlösung von der festen Phase eluiert wird und das Protein oder Peptid anschließend durch Ultrafiltration von den viralen Verunreinigungen abgetrennt wird.

13. Verfahren zur Herstellung eines Proteins oder Peptids mit Hilfe rekombinanter Techniken oder der Hybridoma-Technik durch Kultivierung von Zellen in einem geeigneten Kulturmedium unter Bedingungen, die für die Produktion des gewünschten Proteins oder Peptids durch die Zellen geeignet sind, wobei dem Kulturmedium entweder Serum oder statt dessen gereinigte lipidhaltige Serumkomponenten oder definierte Lipid-Gemische zugegeben werden, das Protein oder Peptid entweder in das Kulturmedium exprimiert oder nach der Kultivierung aus den Zellen freigesetzt wird und durch mehrere Reinigungsschritte aus dem resultierenden Lipide und das Protein oder Peptid enthaltenden Medium gewonnen und in lipidfreiem Zustand isoliert wird, wobei das Protein oder das Peptid in mindestens einem der Reinigungsschritte einer nach Teilchengröße trennenden Filtrationstechnik unterworfen wird, dadurch gekennzeichnet, daß vor der Durchführung der letztgenannten Filtrationstechnik die Lipide von dem Protein oder Peptid abgetrennt werden und das Protein oder Peptid durch ein Reinigungsverfahren gemäß einem der Ansprüche 1 bis 11 isoliert wird.

14. Verfahren gemäß einem der Ansprüche 1 bis 13, dadurch gekennzeichnet, daß das zu reinigende Protein ein Immunglobulin der Klassen A, D, E oder G, ein Interferon, r-tPA oder ein Fab-Fragment ist.

Anhang:

Figur 1:

S-Sepharose FF (Kationenaustauscherchromatographie)

Extinktion bei 280 nm

Beladung —

Waschung mit 2-Propanol —

Elution —

Regeneration —

Zeit

**Figur 2:**

Protein G Sepharose FF (Affinitätschromatographie)

12

Figur 3:

300 KD-Ultrafiltration

Extinktion
bei 280 nm

Zeit

(a)

Extinktion
bei 280 nm

Zeit

(b)

a) nach Lipid-Entfernung

b) ohne vorherige Lipidentfernung

Figur 4:

Ultrafiltrationen nach vorheriger Lipid-Entfernung

a)   300 KD

Extinktion
bei 280 nm

Zeit

b)  200 KD

Extinktion
bei 280 nm

Zeit

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | EP-A-0 197 554 (ARMOUR PHARMACEUTICAL COMPANY) 15. Oktober 1986 * Seiten 1-10; Anspruche1,2,10,12,15 * | 1,6-11, 14 | C07K1/14 C07K3/18 B01D15/08 |
| D,Y | * Insgesamt * | 1-14 | |
| Y | EP-A-0 366 946 (NEW YORK BLOOD CENTER) 7. Oktober 1988 * Insgesamt, besonders Seite 4, Zeile 55 bis Seite 5, Zeile 4 * | 1-14 | |
| X | J. BIOL. CHEM. Bd. 251, Nr. 21 , 10. November 1976 Seiten 6630 - 6637 FILLINGAME, R.H. 'Purification of the carbodiimide-reactive protein component of the ATP energy-transducing system of Echerichia coli' * Seite 6632, Absatz "Chromatography on DEAE-cellulose" * | 1 | |
| X | HARRIS, E.L.V. & ANGAL, S. 'Protein purification applications, a practical approach' 1990 , IRL PRESS , OXFORD * Kapitel 2.2.1 (Seiten 63-65), insbesondere S. 65, 2. und 3. Abschnitt * | 1 | RECHERCHIERTE SACHGEBIETE (Int.Cl.5) C07K B01D B01J |

Der vorliegende Recherchenbericht wurde für alle Patentansprüche erstellt

| Recherchenort | Abschlußdatum der Recherche | Prüfer |
|---|---|---|
| MÜNCHEN | 25. April 1994 | Hermann, R |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
...........................................................................................
& : Mitglied der gleichen Patentfamilie, übereinstimmendes
    Dokument

EPO FORM 1503 03.82 (P04C03)